(19)

Europäisches
Patentamt
European
Patent Office
Office européen
des brevets

(11) **EP 3 327 124 A1**

(12) **EUROPEAN PATENT APPLICATION**

(43) Date of publication:
**30.05.2018 Bulletin 2018/22**

(51) Int Cl.:
*C12N 15/113* [(2010.01)]    *C12N 15/864* [(2006.01)]

(21) Application number: **16200978.1**

(22) Date of filing: **28.11.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Deutsches Krebsforschungszentrum
69120 Heidelberg (DE)**

(72) Inventors:
- **MARCHINI, Antonio
  69120 Heidelberg (DE)**
- **Li, Junwei
  69151 Neckargemund (DE)**

- **ROMMELAERE, Jean
  69118 Heidelberg (DE)**
- **LEUCHS, Barbara
  69121 Heidelberg (DE)**
- **Li-Weber, Min
  69115 Heidelberg (DE)**
- **Nieto, Karen
  69120 Heidelberg (DE)**
- **Krammer, Peter
  69120 Heidelberg (DE)**
- **De-Oliveira, Anna-Paula
  69115 Heidelberg (DE)**

(74) Representative: **Schüssler, Andrea
Kanzlei Huber & Schüssler
Truderinger Strasse 246
81825 München (DE)**

(54) **H-1 PV EXPRESSING RNAI EFFECTORS TARGETING CDK9**

(57)    The present invention relates to innovative protoparvoviruses (PV) expressing RNAi effectors, preferably shRNAs, against the CDK9 gene which display improved anticancer activity. These new viruses are based on the ΔH-1PVsilencer platform that consists of a protoparvovirus H-1PV featuring an in-frame deletion within the NS region (ΔH-1PV) and harbouring a shRNA expression cassette in which the expression of the shRNA is controlled by the H1 Polymerase III promoter. In this invention the inventors aimed to use the ΔH-1PVsilencer to silence the CDK9 gene whose activity is often dysregulated in cancer cells and known to contribute to tumorigenesis. The present invention also provides cells or organisms comprising said parvovirus.

**Description**

The present invention relates to innovative protoparvoviruses (PV) expressing RNAi effectors, preferably shRNAs, against the CDK9 gene which display improved anticancer activity. These new viruses are based on the ΔH-1PVsilencer platform that consists of a protoparvovirus H-1PV featuring an in-frame deletion within the NS region (ΔH-1PV) and harbouring a RNA expression cassette, preferably a shRNA cassette, in which the expression of the RNA is controlled by the H1 Polymerase III promoter. In this invention the inventors aimed to use the ΔH-1PVsilencer to silence the CDK9 gene whose activity is often dysregulated in cancer cells and known to contribute to tumorigenesis. The present invention also provides cells or organisms comprising said parvovirus.

**Background of the Invention**

*RNAi interference, RNAi effectors and delivery*

RNA interference (RNAi) was first recognized in *Caenorhabditis elegans* by Andrew Fire and Craig Mello, who later were awarded with the Nobel prize in 1998 for their discovery. The mechanism of RNAi is based on the sequence-specific degradation of host mRNAs by means of double-stranded RNAs complementary to the target sequence. RNAi is a naturally occurring cellular process that controls gene expression and therefore plays a pivotal role in many cellular processes, including development [1]. It is also a vital component of the immune response defending the cells against pathogens like viruses and transposons [2]. Soon after its discovery, RNAi technology was harnessed to address biological questions and treatment options for diseases owing its highly specificity and capacity to achieve potent knockdown of known genetic sequences [3]. Therapeutically, RNAi works via delivery of small RNA duplexes, including microRNA (miRNA) mimics, small interfering RNAs (siRNAs), short hairpin RNAs (shRNAs) and Dicer substrate RNAs (dsiRNAs) [4]. All the four classes of RNAi effectors are presently tested in a number of phase I-III clinical trials against various diseases including multiple types of cancer [5].

Examples of genes targeted by RNAi-mediated anticancer therapy are *KRAS, Polo-like kinase 1, Furin, Ephrin type-A receptor* and *c-myc.* Most of the clinical studies involve siRNAs conjugated to nanoparticles (e.g. lipid nanoparticles) which have superior stability in comparison to naked siRNAs. However despite significant improvements, a rapid decline of the siRNA is observed within the first 72 h post-injection with most of the siRNA taken up by hepatocytes (approximately 50% of the injected dose is found in the liver already after 30 min from injection). Thus, efficient delivery of siRNAs remains the major bottleneck in this field [5]. As most delivery systems are transient and intracellular concentration of the siRNAs is diluted during cell division, repeated administration of the siRNAs is often required.

Short hairpin RNAs (shRNAs) are another class of RNAi effectors [6]. shRNAs typically consist of two complementary (sense and antisense) 19-29 base pairs sequences separated by a short loop of 4-11 impaired nucleotides. Typically, expression is controlled by a RNA polymerase (Pol) III promoter (*e.g* U6, H1) or modified pol II promoters. After transcription of the shRNA, sense and antisense strand, connected by the loop, pair together forming the characteristic hairpin structure. This structure resembles the pre-miRNA that are naturally used by the cell to regulate gene expression and requires nuclear processing [3]. After the discovery of promoter-driven expression of shRNAs, the design of viral RNAi vectors became possible [7]. Examples of viruses employed for the delivery and expression of shRNAs in cancer gene therapy are adenoviruses, adeno-associated viruses, lentiviruses and retroviruses. Most of these viruses, however, are defective in replication and therefore the silencing effect is restricted to the primary infected cells.

*Oncolytic viruses and RNAi*

Oncolytic viruses (OVs) are viruses which selectively replicate in and kill cancer cells and have the ability to spread throughout the tumour while sparing normal tissues. Their anticancer potential has been demonstrated at the preclinical level against a vast variety of tumour models and at least twelve OVs are presently under phase I-III clinical evaluation. In particular, Talimogene laherparepvec (T-Vec; Amgen), a modified herpes simplex virus (HSV) expressing the immunostimulatory cytokine granulocyte-macrophage colony-stimulating factor (GM-CSF), has been approved recently by FDA and EMA for the treatment of unresectable metastatic melanoma. However, the promising results obtained with OVs at the preclinical levels have not always been reproduced in clinic. Tumours are often highly heterogeneous in nature and it may be that certain tumours are moderately susceptible to virus-induced oncolysis. It is also possible that within a certain tumour a fraction of cells survives virus treatment leading to tumour re-growth. The application of RNAi technology in the context of OV-based therapy is therefore very attractive because it may represent a mean to reinforce the efficacy of OVs by providing the virus an additional mode of action for killing those cancer cells that are poorly sensitive to its infection. In addition, OV-mediated delivery of RNAi effectors may overcome the general hurdle of RNAi technology how to achieve high-level expression of these molecules specifically in cancer cells, in particular after intravenous administration. Furthermore, OV can replicate and spread through the tumour thereby having the potential to amplify shRNA production and delivery. Arming OVs with RNAi effectors proven to be a valid approach in the case of oncolytic adenoviruses (Ad) e.g. Ad expressing shRNAs targeting a number of tumour related genes including *VEGF, MYCN, SATB1, C-Met, Ki 67, IL-8, hTERT and FAK* have superior anticancer activity than their parental viruses [8-15]. It was also shown that oncolytic HSV can be engineered to efficiently express RNAi effectors (shRNAs and artificial miRNAs) [16].

**Object of the Present Invention**

As mentioned above, if shRNA is delivered by adenoviruses, adeno-associated viruses, lentiviruses and retroviruses, the viruses are often defective in replication and therefore the silencing effect is restricted to the primary infected cells.

Therefore, it is the object of the present invention to provide means for efficiently down regulating the expression of cancer-related genes in a cell or organism in a more durable way.

According to the invention this is achieved by providing the subject matters defined in the claims.

**Description of the present Invention**

The present invention concerns a deletion variant of autonomous parvovirus H-1 expressing a RNAi effector, preferably shRNAs, against the CDK9 gene which is a cancer-related gene.

H-1 PV have attracted high attention for their anti-cancer potential because they are non pathogenic for humans and possess oncolytic and oncosuppressive properties. Pre-existing anti-viral immunity is usually not a problem for these viruses as humans are normally not exposed to rodent parvovirus infection. The parvovirus genome consists of a single stranded DNA of approximately 5100 bases containing two promoters, P4 and P38 which regulate the expression of the non-structural (NS1 and NS2) and capsid (VP1 and VP2) proteins, respectively. Activation of the P4 promoter is a critical step in the PV life cycle. The activity of the P4 promoter is later modulated by its own gene product NS1, but its initial activation is completely dependent on host cellular factors, which are mainly expressed during the S phase of the cell cycle. This dependency, together with the fact that the virus is unable to stimulate quiescent cells to proliferate, contributes to the oncotropism of the virus, which replicates preferentially in proliferating, transformed or malignant cells. In addition, parvovirus cytotoxicity is also stimulated by cellular changes associated with neoplastic transformation. NS1 is the major viral toxic protein. H-1PV has been shown to activate several death pathways in cancer cells. Although the anticancer potential of PVs is supported by a large set of preclinical studies, efficacy can be expected to be a limiting factor in clinical applications. It is possible that some cancer cells survive virus treatment, causing tumour relapse.

During the experiments resulting in the present invention it could be shown that the insertion of an shRNA expression cassette targeting the CDK9 gene at a particular site of the parvovirus genome is compatible with parvovirus packaging capacity, does not interfere with viral replication and enhances the intrinsic cytotoxicity of the virus. The virus expresses high levels of shRNAs and is very efficient in gene silencing. The big advantage of H-1PV-silencer in comparison with replication defective vectors resides in its capacity to replicate and propagate in proliferating cells, e.g., cancer cells. Every infected/transduced cell theoretically could become a producer of novel viral particles. Progeny virions of through second rounds of infection could spread through the tumour and efficiently delivery and express therapeutic shRNAs. In this setting, the silencing signal could be amplified beyond the initial inoculum. Together, parvovirus-based vectors and shRNA technology compensate each other limitations: the natural oncotropism of parvovirus should specifically and effectively deliver to and mediate transduction of the shRNAs in proliferating cells, e.g., cancer cells.

**Brief description of the drawings**

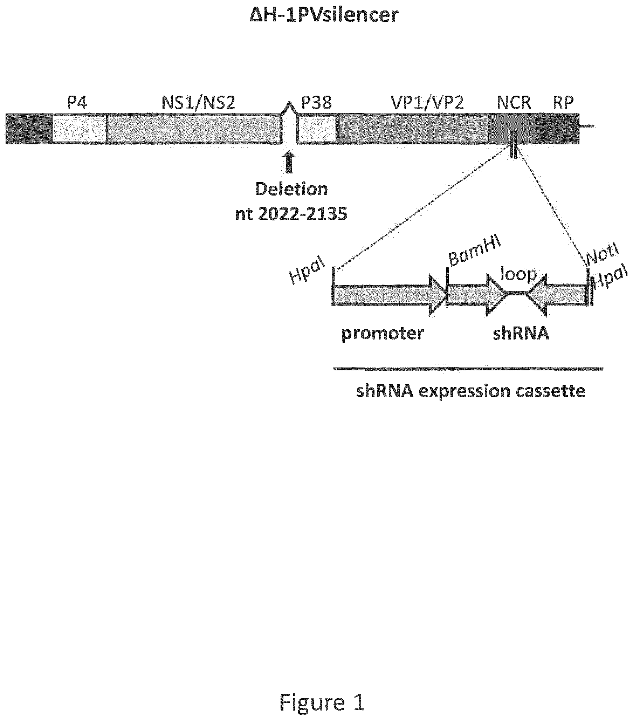

**Figure 1: Schematic representation of the H-1PV silencer vector.**
The ΔH-1PV virus genome features an in frame deletion encompassing nucleotides (nt) 2022 - 2135. The left (LP) and right palindromic sequence (RP) serve as self-priming origins of replication; P4 promoter regulates the expression of the NS gene, encoding the nonstructural proteins NS1 and NS2, and P38 promoter regulates the expression of the VP gene encoding the VP1 and VP2 viral proteins. The non-coding region (NCR) is downstream the VP region and it is supposed to be involved in the regulation of virus genome replication and encapsidation. The shRNA expression cassette, consisting of a H1-PolIII promoter and a targeting shRNA sequence, is inserted into the *Hpa*I restriction site within the NCR of the ΔH-1PV genome.

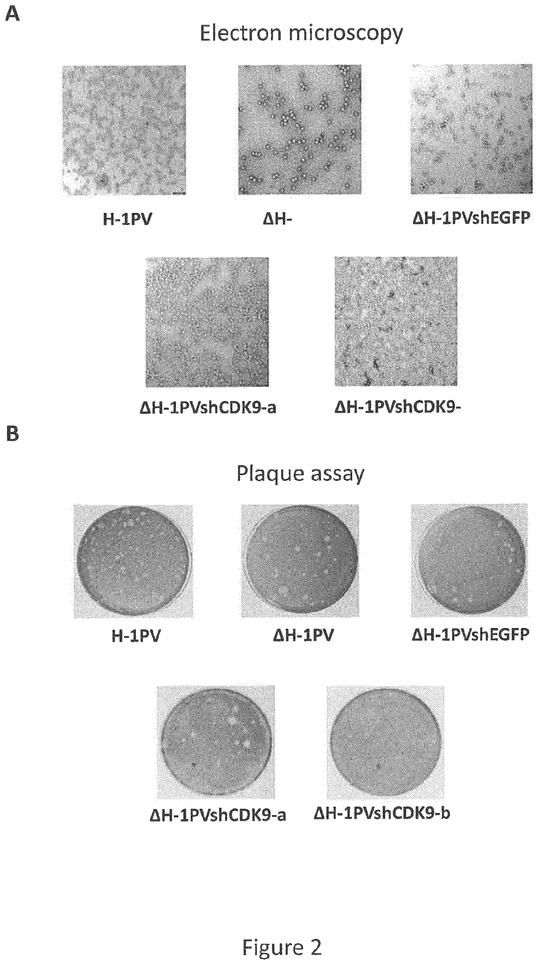

**Figure 2: Transfection of pΔH-1PVshCDK9 in HEK293T cells results in generation of fully infectious virus particles.**

**A)** Morphological phenotype. Viruses produced in HEK293T cells were analyzed by electron microscopy as described in the Materials and Methods section. No differences in morphology among the various viruses were found.

**B)** Comparison of plaque sizes. 10 fold-serial dilutions of indicated viruses were used for plaque assay in NB324K indicator cells as described in the Materials and Methods section. Staining of the plaques was carried out 5 days post-infection for all viruses with the exception of ΔH-1PVshCDK9-b for which staining was carried out after 6 days.

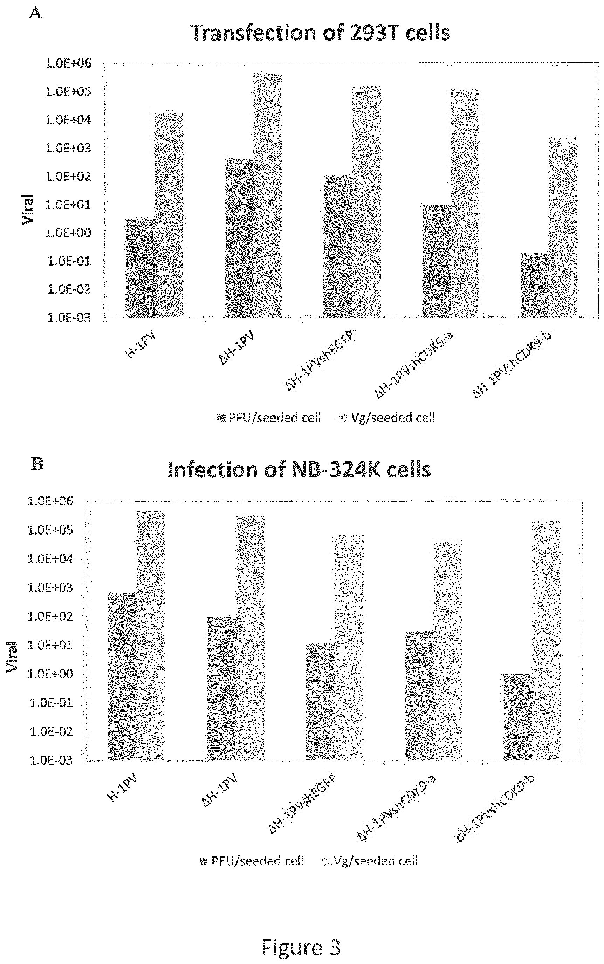

## Figure 3. ΔH-1PVshCDK9 can be produced at high titers.

**A)** Production in HEK293T cells via transfection. DNA plasmids harboring virus genomes were transiently transfected in HEK293T cells. After virus purification, virus batches were titrated by qPCR and plaque assay. Values obtained by qPCR are expressed as encapsidated viral genome (Vg) per seeded cells while those obtained by plaque assay are expressed as plaque forming unit (PFU) per seeded cells.

**B)** Production in NB324K cells via infection. NBK324 cells were infected with the indicated viruses at the same MOI (0.1 PFU/cell). Produced viruses were then purified and titrated as described above.

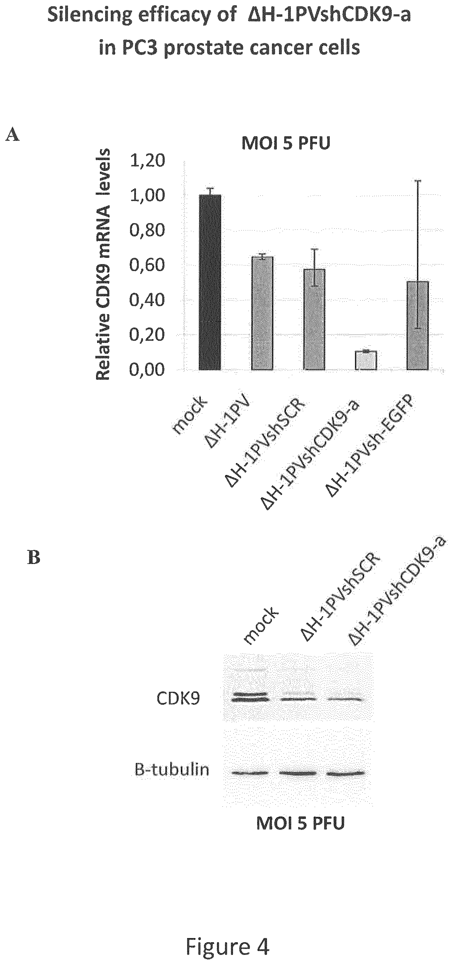

## Figure 4. ΔH-1PVshCDK9 is efficient in gene silencing.

**A)** PC3 cells were infected with MOI 5 (PFU/cell) of the indicated viruses. Cells were harvested at 48 h post-infection and total RNA was isolated and reverse transcribed. *CDK9* mRNA levels were quantified by qRT-PCR using specific primers. Values were normalized with *GAPDH* (used as housekeeping gene). Values are expressed as percentage relative to values obtained in mock-treated cells with standard deviation bars. Mean values from a representative experiment performed in triplicate are presented.

**B)** PC3 cells were infected with MOI 5 (PFU/cell) of the indicated viruses and grown for 72 h before to be lysed. Total cell extracts were subjected to SDS-PAGE followed by immunoblot analysis of the protein levels of CDK9 and β-tubulin (loading control) using specific antibodies.

## Figure 5. ΔH-1PVshCDK9 has superior oncolytic activity (I)

PC3 cells were infected with the indicated viruses at MOI (PFU/cell)1 (upper panel) or 5 (lower panel). At 72 h post infection, cell membrane integrity was assayed using the ApoTox-GloTM Triplex Assay kit according to the protocol described in the Materials and Methods section.

## Figure 6. ΔH-1PVshCDK9 has superior oncolytic activity (II)

PC3 cells were infected with the indicated viruses at MOI (PFU/cell)1 (upper panel) or 5 (lower panel). After 72, 96, or 120 h, cells were harvested, fixed and processed for flow cytometry as described in the Materials and Methods section by staining the cells with propidium iodide and analyzing the apoptotic subG1 cell population containing fragmented DNA.

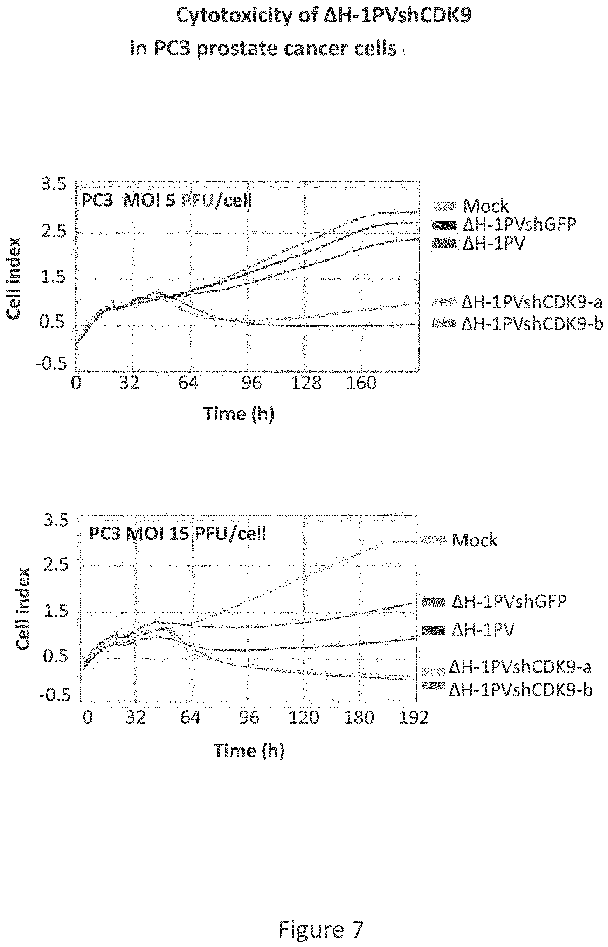

## Figure 7. ΔH-1PVshCDK9 has superior oncolytic activity (III)

PC3 cancer cells were seeded in 96-well E-plates, and infected with the indicated viruses at MOI 1 (PFU/cell) (upper panel) or MOI 15 (lower panel) or treated with buffer (mock). Cell proliferation and viability were monitored in real-time with the XCELLigence system every 30 min for approximately 7 days. Values are expressed as normalized cell index which reflects the number of viable cells attached at the bottom of the well. Cell indexes are plotted against the time as a representation of cell growth curves. Each experiment condition was performed at least in triplicates and average values are shown. The arrow indicates the time of treatment (approximately 24 h after cell plating).

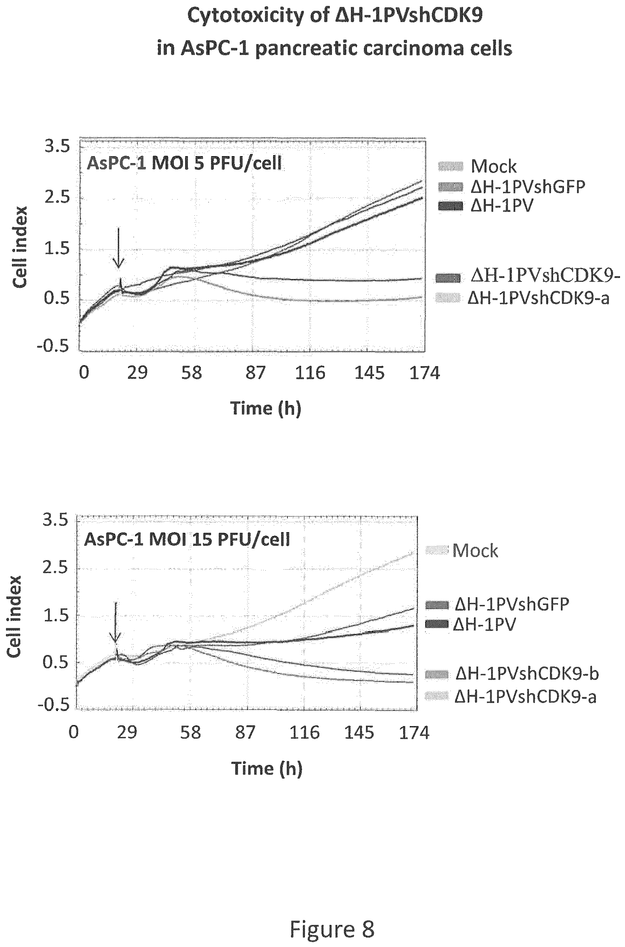

## Figure 8. ΔH-1PVshCDK9 has superior oncolytic activity (IV)

AsPC-1 were seeded in 96-well E-plates, and infected with the indicated viruses at MOI 1 (PFU/cell) (upper panel) or MOI 15 (lower panel) or treated with buffer (mock). Cell proliferation and viability were monitored in real-time with the XCELLigence system as described in Figure 7. The arrow indicates the time of treatment (approximately 24 h after cell plating).

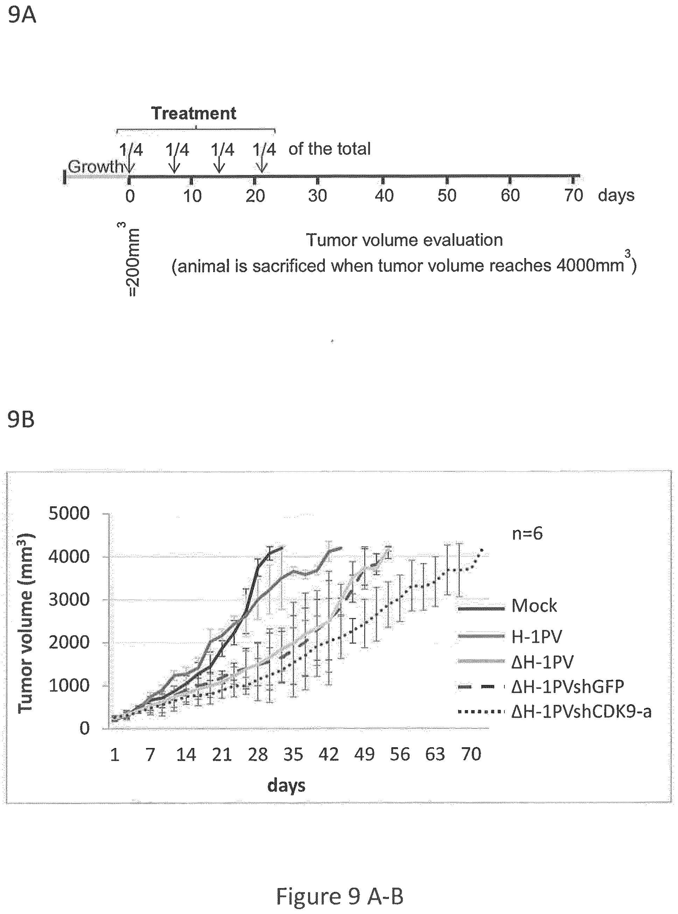

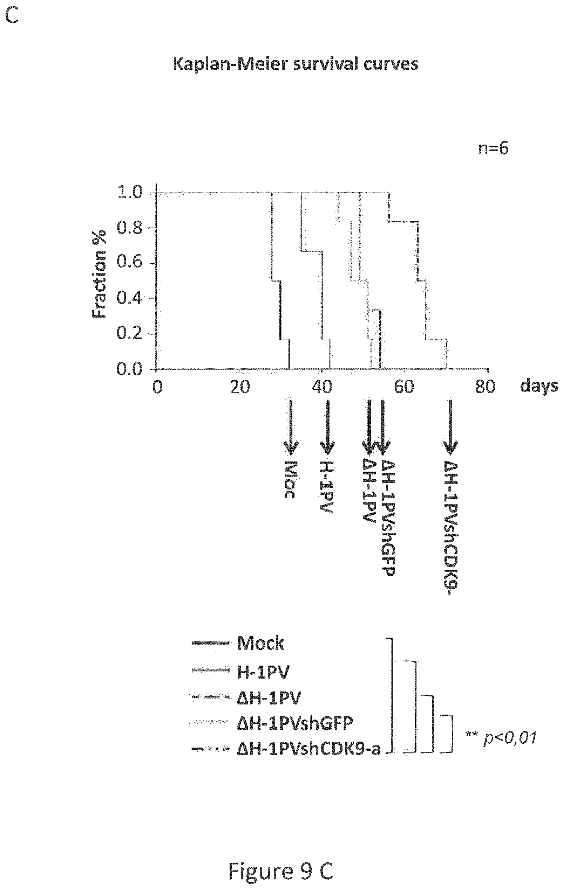

## Figure 9. ΔH-1PVshCDK9 has enhanced oncosuppressive capacity.

**A)** Experimental schedule. $5x10^6$ pancreatic carcinoma derived AsPC-1 cells were subcutaneously injected in the right flank of 5 week-old female nude rats. After 7-10 days (when tumour reached the volume of 200-250 mm3), tumour-bearing animals were randomized into four groups (n=6). Groups were treated either with PBS (control), or virus (total dose of $1 x 10^8$ pfu/animal, fractionated in 4 intratumoral administrations).

B) Tumour growth. Tumour volume was measured with a digital calliper on the days indicated and calculated according to the formula: volume ($cm^3$) = $width^2$ x length/2. Rats were sacrificed when the tumour mass reached 4000 $mm^3$, in keeping with animal welfare regulations. Data shown represents the average values with standard deviation bars.

C) Survival curves. Survival of the tumour bearing rats treated as indicated was analysed using the Kaplan-Meier method. Animals were arbitrarily considered as dead when tumour was reaching the size of 4000 $mm^3$. Statistical significance in survival rates was calculated using the two sided log-rank test for significance and adjusted for multiple testing with the Bonferroni method. \*\*P < 0.01 was considered statistically significant.

**Detailed Description of the Present Invention**

Thus, the present invention provides a parvovirus based on a parvovirus H-1 deletion variant for down regulating the expression of CDK9 in a cell characterized in that the parvovirus H-1 deletion variant contains a deletion encompassing the nucleotides 2022-2135, wherein the CDK9 specific nucleic acid is inserted in an untranslated region downstream of the H-1 parvovirus VP gene and is expressible under the control of a promoter or promoter region recognizable by an RNA polymerase in the cell, wherein said CDK9 specific nucleic acid is transcribable in an RNAi, and wherein said parvovirus is capable of replicating and propagating in the cell.

The parvovirus H-1 deletion variant (ΔH-1PV) features an in-frame deletion encompassing nucleotides (nt) 2022 - 2135 of wildtype parvovirus H-1 (Fig. 1). The left (LP) and right palindromic sequence (RP) serve as self-priming origins of replication; P4 promoter regulates the expression of the NS gene, encoding the nonstructural proteins NS1 and NS2, and P38 promoter regulates the expression of the VP gene encoding the VP1 and VP2 viral proteins. The non-coding region (NCR) is downstream the VP region and it is supposed to be involved in the regulation of virus genome replication and encapsidation.

Cyclin-dependent kinase 9 (CDK9) is a cyclin-dependent kinase associated with P-TEfb. The protein encoded by this gene is a member of the cyclin-dependent kinase (CDK) family. CDK family members are known as important cell cycle regulators. This kinase was found to be a component of the multiprotein complex TAk/P-TEFBb which is an elongation factor for RNA polymerase II-directed transcription and functions by phosphorylating the C-terminal domain of the largest subunit of RNA polymerase II. CDK9 is involved in AIDS by interaction with HIV-1 Tat protein, in differentiation of skeletal muscle and plays in a role in cancerogenesis.

The CDK9 specific nucleic acid is inserted into the viral genome in such a way that viral replication and cytotoxicity are not affected, i.e. downstream of the parvovirus VP gene encoding the capsid proteins of the parvovirus. Preferably, the CDK9 specific nucleic acid is inserted at nucleotide 4683 of the H-1PV genome.

The term "CDK9 specific nucleic acid" as used herein refers to a nucleic acid comprising at least 15, 20, 25, 50, 100 or 200 consecutive nt having at least about 75%, particularly at least about 80%, more particularly at least about 85%, quite particularly about 90%, especially about 95% sequence identity with the complement of a transcribed nucleotide sequence of the CDK9 target gene.

In the present invention the CDK9 gene can be down regulated in an *in vivo* cell or an *in vitro* cell (ex vivo). The cell may be a primary cell or a cell that has been cultured for a period of time or the cells may be comprised of a cultured cell line. The cell may be a diseased cell, such a cancer cell or tumor or a cell infected by a virus. The cell may be a stem cell which gives rise to progenitor cells, more mature, and fully mature cells of all the hematopoietic cell lineages, a progenitor cell which gives rise to mature cells of all the hematopoietic cell lineages, a committed progenitor cell which gives rise to a specific hematopoietic lineage, a T lymphocyte progenitor cell, an immature T lymphocyte, a mature T lymphocyte, a myeloid progenitor cell, or a monocyte/macrophage cell. The cell may be a stem cell or embryonic stem cell that is omnipotent or totipotent. The cell maybe a nerve cell, neural cell, epithelial cell, muscle cell, cardiac cell, liver cell, kidney cell, stem cell, embryonic or foetal stem cell or fertilised egg cell.

Preferably, said parvovirus variant is formulated as a pharmaceutical composition, wherein the parvovirus is present in an effective dose and combined with a pharmaceutically acceptable carrier.

"Pharmaceutically acceptable" is meant to encompass any carrier which does not interfere with the effectiveness of the biological activity of the active ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc.. Additional pharmaceutically compatible carriers can include gels, bioasorbable matrix materials, implantation elements containing the parvovirus (therapeutic agent), or any other suitable vehicle, delivery or dispensing means or material(s). Such carriers can be formulated by conventional methods and can be administered to the subject at an effective dose.

An "effective dose" refers to amounts of the active ingredients that are sufficient to effect treatment. An "effective dose" may be determined using methods known to one skilled in the art (see for example, Fingl et al., The Pharmocological Basis of Therapeutics, Goodman and Gilman, eds. Macmillan Publishing Co., New York, pp. 1-46 ((1975)).

Administration of the parvovirus may be effected by different ways, e.g. by intravenous, intratumoral, intraperetoneal, subcutaneous, intramuscular, topical or intradermal administration. The route of administration, of course, depends on the kind of therapy. Preferred routes of administration are intravenous (i.v.), intratumoral or endobronchial administration. If infectious virus particles are used which have the capacity to penetrate through the blood-brain barrier, treatment could be performed or at least initiated by intravenous injection of, e.g., H1 virus.

The dosage regimen of the parvovirus is readily determinable within the skill of the art, by the attending physician based an patient data, observations and other clinical factors, including for example the patient's size, body surface area, age, sex, the particular modified parvovirus etc. to be administered, the time and route of administration, the type of mesenchymal tumor, general health of the patient, and other drug therapies to which the patient is being subjected.

As another specific administration technique, the parvovirus can be administered to the patient from a source implanted in the patient. For example, a catheter, e.g., of silicone or other biocompatible material, can be connected to a small subcutaneous reservoir (Rickham reservoir) installed in the patient, e.g., during tumor removal, or by a separate procedure, to permit the parvovirus to be injected locally at various times without further surgical intervention. The parvovirus can also be injected into a tumor by stereotactic surgical techniques or by neuronavigation targeting techniques.

Administration of the parvovirus can also be performed by continuous infusion of viral particles or fluids containing viral particles through implanted catheters at low flow rates using suitable pump systems, e.g., peristaltic infusion pumps or convection enhanced delivery (CED) pumps.

As yet another method of administration of the parvovirus is from an implanted device constructed and arranged to dispense the parvovirus to the desired tissue. For example, wafers can be employed that have been impregnated with the parvovirus, e.g., parvovirus H1, wherein the wafer is attached to the edges of the resection cavity at the conclusion of surgical tumor removal. Multiple wafers can be employed in such therapeutic intervention. Cells that actively produce the parvovirus H1 variant, can be injected into the tumor, or into the tumor cavity after tumor removal.

In a particularly preferred embodiment of the present invention, the target specific nucleic acid is inserted at the HpaI restriction site of the H-1PV genome. However the insertion of the cassette in other regions of parvovirus genome is also considered as well as other RNAi triggering molecules such as microRNAs and/or antisense oligonucleotides. In a further particularly preferred embodiment of the present invention, the promoter or promoter region recognizable by RNA polymerases is a RNA-polymerase II (Pol II) promoters such as for instance CMV and human ubiquitin C or RNA-polymerase III (Pol III) promoters such as U6, H1, 7SK and tRNA. An example of a particularly preferred RNA-polymerase III (Pol III) promoter is the RNA-polymerase III H1 promoter.

In a preferred embodiment of the present invention the CDK9 specific nucleic acid is an shRNA. An shRNA is a small hairpin RNA or short hairpin RNA that is a sequence of RNA that makes a tight hairpin turn that can be used to silence gene expression via RNA interference. The shRNA hairpin structure is cleaved by the cellular machinery into siRNA, which is then bound to the RNA-induced silencing complex (RISC). This complex binds to and cleaves mRNAs which match the siRNA that is bound to it.

In a further particularly preferred embodiment of the present invention, the CDK9 specific nucleic acid, e.g., shRNA, has a length of at least 15 nucleotides. In a particular preferred embodiment the CDK9 sequence contains nt235-253 of sequence ID:XM_017014184.1. In another preferred embodiment the CDK9 sequence contains nt594-614 of sequence ID:XM_017014184.1. Since the shorter sequence (nt 235-253) showed better infectivity (Fig. 3B) than the longer sequence, the shorter sequence is slightly preferred.

The present invention also relates to a rodent parvovirus as characterized above for use in treating cancer.

In a preferred embodiment, said parvovirus can be used for treating a tumour, in particular (but not exclusively) prostate cancer, pancreatic carcinoma, brain cancer (preferably gliomas), cervical carcinoma, lung cancer, head and neck cancer, breast cancer or colon cancer.

In a further preferred embodiment, said parvovirus can be used for the treatment of a tumour characterized in that the cells of said tumour are resistant to chemotherapy and/or radiotherapy.

Patients treatable by the parvovirus according to the invention include humans as well as non-human animals. Examples of the latter include, without limitation, animals such as cows, sheep, pigs, horses, dogs, and cats.

The present invention also provides a cell of an animal, fungus or protist comprising a parvovirus as hereinbefore described. In an embodiment, the cell is *in vitro.* The cell is preferably an animal cell, an isolated human cell, an *in vitro* human cell, a non-human vertebrate cell, a non-human mammalian cell, fish cell, cattle cell, goat cell, pig cell, sheep cell, rodent cell, hamster cell, mouse cell, rat cell, guinea pig cell, rabbit cell, non-human primate cell, nematode cell, shellfish cell, prawn cell, crab cell, lobster cell, insect cell, fruit fly cell, Coleapteran insect cell, Dipteran insect cell, Lepidopteran insect cell or Homeopteran insect cell.

Finally, the present invention also provides a transgenic, non-human animal, fungus or protist comprising a parvovirus as hereinbefore described. Transgenic animals can be produced by the injection of the parvovirus into the pronucleus of a fertilized oocyte, by transplantation of cells, preferably uindifferentiated cells into a developing embryo to produce a chimeric embryo, transplantation of a nucleus from a recombinant cell into an enucleated embryo or activated oocyte and the like. Methods for the production of trangenic animals are well established in the art and, e.g., described in US patent 4,873, 191.

In summary, the new viruses are based on the ΔH-1PVsilencer platform that consists of a protoparvovirus H-1PV featuring an in frame-deletion within the NS region (ΔH-1PV) and harbouring an RNA expression cassette, preferably a shRNA expression cassette, in which the expression of the shRNA is controlled by the H1 Polymerase III promoter. The ΔH-1PVsilencer is effective in gene silencing while keeping its ability to replicate and be fully infectious. In the present invention the ΔH-1PVsilencer was used to silence the CDK9 gene whose activity is often dysregulated in cancer cells and known to contribute to tumorigenesis. Two different shRNA sequences targeting different regions of the CDK9 gene were designed and inserted in pΔH-1PVsilencer vector resulting in the construction of pΔH-1PVsh-CDK9a and pΔH-1PVsh-CDK9b plasmids. Transfection of the plasmids in HEK293T cells generated fully infectious viral particles that can be further amplified via infection in NB324K cells following a classical parvovirus production protocol. Remarkably, viruses expressing shRNAs targeting CDK9 showed superior oncolytic activities in comparison with the parental ΔH-1PV and the wild type H-1PV in two cell lines derived from prostate (PC3) or pancreatic (AsPC-1) carcinomas (Fig. 9). The enhanced cell killing was associated with virus-mediated down-regulation of the CDK9 gene. Validation of these results in a xenograft rat model of human pancreatic carcinomas confirmed the improved anticancer activity of parvoviruses expressing shRNAs targeting CDK9.

The below examples explain the invention in more detail.

**Example 1**

**Materials and Methods**

Plasmid construction and viruses

The pΔH-1PVsilencer, pΔH-1PVshSCR (containing a scramble shRNA), pΔH-1PVshEGFP (with a shRNA targeting EGFP) vectors have been previously described [19]. pΔH-1PVsilencer contains a H-1PV viral genome featuring:

(i) an in-frame deletion of 114 nucleotide (extending from nucleotide 2022 to 2135 according to the NCBI gene bank reference sequence X01457.1 ) within the NS coding region [18];

ii) a shRNA expression cassette inserted at the unique *Hpa*I (GTTAAC) restriction enzyme site of the H-1PV genome (corresponding to position 4686- 4691 of the wild type virus) which allows the virus to express high levels of shRNAs while keeping its ability to replicate and propagate autonomously (Fig. 1). The shRNA expression cassette contains *BamH*I and *Not*I restriction enzyme sites for easy cloning of the shRNA sequence using overlap oligonucleotides directly ligated into a previously *BamH*I/*Not*I digested plasmid. The oligonucleotides that were used in this study for the generation of pΔH-1PVshCDK9a and pΔH-1PVshCDK9b were (shRNA loop sequence underlined):

Cdk9a sense (matching CDK9 sequence 235-253 of sequence ID:XM_017014184.1)

5'–

GATCCGTGAGATTTGTCGAACCAAATTTCAAGAGAATTTGGTTCGACAAATCTCATTTTTTG

GAAGC-3'

Cdk9a antisense:

5´-GGCCGCTTCCAAAAAATGAGATTTGTCGAACCAAATTCTCTTGAAATTTGGTTCGA

CAAATCTCACG-3´

Cdk9b sense (matching CDK9 sequence 594-614 of sequence ID:XM_017014184.1)

5´-GATCCGCTACTACATCCACAGAAACAATTCAAGAGATTGTTTCTGTGGATGTA

GTAGTTTTTTGGAAGC-3´

Cdk9b antisense:

5´-GGCCGCTTCCAAAAACTACTACATCCACAGAAACAATCTCTTGAATTGTTTC

TGTGGATGTAGTAGCG-3´

### Virus production

HEK293T cells were seeded at a density of 5.5 x $10^5$ cells/dish in 22 cm$^2$ dishes in 10 ml medium (TPP, Trasadingen, Switzerland). On the next day, cells were transiently transfected with 15 µg of plasmids harbouring the virus genome (pΔH-1PV, pΔH-1PVshSCR, pΔH-1PVshEGFP, pΔH-1PVshCDK9-a or pΔH-1PVshCDK9-b) using the transfection reagent X-tremeGene™ (Roche Diagnostics, Mannheim, Germany) at 1:2 ratio (µg DNA: µl reagent), according to manufacturer's instructions. Cells were harvested 3 days post-transfection and cell-associated virus particles were released in VTE buffer (50 mM Tris pH8.7, 0.5 mM EDTA) through three freeze-thaw cycles. Cellular debris was removed by centrifugation at 5,000 rpm for 10 min, and crude viral extracts were digested with 50 U/ml Benzonase® Nuclease (Sigma-Aldrich Chemie GmbH, Steinheim, Germany - ultrapure grade) at 37 °C for 30 min to remove non-encapsidated viral genomes.

NB324K cells were used for virus stocks amplification. Cells were seeded in 148 cm$^2$ dishes (TPP, Trasadingen, Switzerland) at a density of 3x$10^6$ cells/dish in 40 ml medium. On the next day, cells were infected with the different virus batches

(previously produced through plasmid transfection in HEK293T cells) at a low multiplicity of infection (MOI) of 0.1 plaque forming unit (PFU/cell), or in some experiments 100 viral genomes (Vg)/cell. After 4 days of infection, when cytopathic effect (CPE) had reached 80% of cell monolayer, cells were harvested and crude viral extracts were prepared as described above.

### Purification of viral particles

Viruses were purified according to Leuchs et al. [26]. Purified virions were aliquoted and stored at -80°C.

### Titration of viral stocks

*a) Plaque assay*

NB324K cells were seeded at a density of 5x$10^5$ cells/dish in 22 cm$^2$ dishes. On the next day, the growth media was removed and cells were infected with 400 µl of serial 10-fold dilutions of virus stocks for 1 h at 37 °C. The inoculum was removed and cells were overlaid with a semi-solid medium consisting of 0.65% Bacto agar (Becton, Dickinson GmbH, Heidelberg, Germany), MEM (2x MEM - Gibco, Invitrogen), 5 % FBS, 2 mM l-glutamine, 100 U/ml penicillin, and 100 µg/ml streptomycin. Cells were incubated for 4 days at 37 °C, 5% $CO_2$. Living cells were stained for 18 h with a neutral red staining solution consisting of 0.2 mg/ml neutral red, 0.85 % Bacto agar in phosphate buffered saline (PBS). Plaques were counted, and titers expressed as plaque forming units (PFU) per seeded cells.

*b) Quantification of viral genomes by qPCR*

Viral DNA was extracted using the QiaAmp MinElute virus kit (Qiagen, Hilden, Germany) according to manufacturer's instructions. Quantification of encapsided viral DNA was carried out by quantitative real-time PCR (qPCR) targeting the NS1 gene of H-1PV. A fragment of 141 nucleotides was amplified with a forward primer: 5'-GCGCGGCAGAATTCAAACT-3', and a reverse primer: 5'- CCACCTGGTTGAGCCATCAT-3', and detected with an NS1-specific TaqMan probe: 5'- 6-FAM-ATGCAGCCAGACAGTTA-MGB-3' (Applied Biosystems, Darmstadt, Germany). A plasmid containing the NS1 sequence was used as a standard in serial 10-fold dilutions in the range of $10^1$-$10^8$ copies for reaction. The reaction mix containing the TaqMan Universal Master Mix (Applied Biosystems, Darmstadt, Germany), 10 pmol/µl of each primer and probe, and 6.7 µl of viral DNA dilutions was run under standard conditions, and the threshold cycle of fluorescence (ct) for each sample was determined by real-time PCR using the Mastercycler EP realplex system (Eppendorf, Hamburg, Germany)[26]. Absolute quantification of viral yields was calculated and expressed as viral genomes (Vg) per seeded cells.

### Electron microscopy

For qualitative analysis of virus preparations, electron microscopy pictures were taken. To this end, 5 µl of 0.05% BSA solution was added to a ready-to-use carbon-coated copper grid (Plano GmbH, Wetzlar, Germany) and incubated for 1 min. The BSA was then removed with Whatman 50 filter paper and the grid was incubated with 5 $\mu$l virus suspension for 2 min and afterwards with 0.1% glutaraldehyde for 5 min. The grid was then washed three times with 5 $\mu$l Bidest water and coated with 2% uranyl acetate for 30 sec. The drops were absorbed from the grid with Whatman 50 filter paper and the grid was dried for approximately 1 min. Images were acquired with a Zeiss EM 900 transmission electron microscope (Carl Zeiss Microscopy GmbH, Jena, Germany) at 85,000 x magnification.

<u>Cell lines</u>

Two human cancer cell lines were selected to test the superior anticancer activity of parvoviruses expressing shRNAs against CDK9, namely AsPC-1, derived from a pancreatic carcinoma and PC3 derived from prostate cancer. These cell lines were selected for the following reasons: (i) they are semi-permissive to wild type H-1PV cytotoxicity (killed only in the presence of high concentrations of the virus) and (ii) our pilot transfection experiments showed that siRNA mediated silencing of CDK9 was effective in inducing cell death (data not shown). All cell lines were obtained from ATCC (LGC Standards GmBH, Wesel, Germany) and grown in Dulbecco's modified Eagle's (DMEM) medium supplemented with 10% foetal bovine serum (FBS) (Sigma-Aldrich Chemie GmbH, Steinheim, Germany), 100 U/ ml penicillin, 100 $\mu$g/ml streptomycin and 2 mM L-glutamine (Gibco, Invitrogen, Karlsruhe, Germany). Cells were grown at 37°C in 5% $CO_2$ and 92% humidity. Cells were periodically checked for mycoplasma contamination and authentication by the Genomic and Proteomics DKFZ core facility.

Quantitative CDK9 mRNA real-time PCR

RNA was isolated using the RNeasy kit (Qiagen, Hilden, Germany) according to the manufacturer's instructions. A 1$\mu$g of total RNA was reverse transcribed using the Perkin Elmer GeneAmp RNA PCR kit (Foster City, CA, USA). The incorporation of the fluorescent DNA-intercalating reporter dye SYBR Green, was detected using the ABI Prism 7500 qRT-PCR cycler (Software: 7500 2.0.3). Master mixes for duplicate measurements were prepared according as follows:

| | |
|---|---|
| Template cDNA | 1 $\mu$l |
| Forward primer (5 $\mu$M) | 5.1 $\mu$l |
| Reverse primer (5 $\mu$M) | 5.1 $\mu$l |
| Power SYBR© Green Master Mix (2x) | 42.5 $\mu$l |
| ddH$_2$O | 30.3 $\mu$l |
| total volume | 85 $\mu$l |

The sequences of primers are the following:

CDK9P42-F 5'-GCCAAGATCGGCCAAGGCAC-3'
CDK9P42-R 5'-CAGCCCAGCAAGGTCATGCTC- 3'
CDK9P55-F 5'-CCTCTGCAGCTCCGGCTCCC- 3'
CDK9P55-R 5'- CACTCCAGGCCCCTCCGCGG-3'

Reactions were carried out in duplicates with a final volume of 25 $\mu$l per reaction in a 96 well plate. The Power SYBR® Green PCR Master Mix contains 300 $\mu$l 10x SYBR Green buffer, 360 $\mu$l 25 mM $MgCl_2$, 240 $\mu$l dNTP(10 mM each), 15 $\mu$l U/$\mu$l Hot Gold Star Taq polymerase and 30 $\mu$l U/$\mu$l uracil-Nglycosylase. Relative gene expression levels were determined through cycle threshold values (Ct) by normalisation to the house-keeping gene glyceraldehyde-3 phosphate (GAPDH) as reference gene. Results are presented as fold expression compared to transcript levels of non infected cells (mock), which were set to 1. Fold expression was calculated using the $\Delta\Delta$Ct method (Pfaffl, 2001) according to the following formula: relative mRNA expression = 2-(Ct of gene of interest - Ct of GAPDH).

<u>Cell lysis and protein quantification</u>

Cells were pelleted by centrifugation and washed once with ice-cold PBS. Subsequently, the pellet was resuspended in 20 $\mu$l of ice-cold lysis buffer (10x buffer; 20 mM Tris-HCI (pH 7.5), 150 mM NaCl, 1 mM $Na_2$EDTA, 1 mM EGTA, 1% Triton X100, 2.5 mM sodium pyrophosphate, 1 mM $\beta$-glycerophosphate, 1 mM $Na_3VO_4$, 1 $\mu$g/ml leupeptin) per 1x10$^6$ cells. Lysates were incubated on ice for at least 20 minutes. Whole cell lysates were cleared from insoluble cell content by centrifugation at 10,000 rpm at 4°C in a table top centrifuge for 15 minutes. Protein concentration of whole cell lysates was determined using the Roti-Quant® Bradford assay (Carl Roth GmbH, Karlsruhe Germany) according to the manufacturers' instructions. Dilutions of 0 to 2 $\mu$g/ml bovine serum albumin in lysis buffer were used as a standard. 1 $\mu$l of sample was mixed with 1 ml of Roti-Quant® Bradford reagent and incubated for 5 min in the dark. After incubation, absorbance at 595 nm was measured in a photometer, and concentrations were determined according to the standard curve.

SDS-PAGE and Immunoblot analysis

For polyacrylamide gel electrophoresis (PAGE), lysates (adjusted to equal protein levels) were mixed with an appropriate amount of 2x or 5x reducing SDS sample buffer [27] and heated to 95°C for 5 min. For separation of proteins, 8-12% polyacrylamide separating gels and 5% polyacrylamide stacking gels were used. Electrophoretic separation was conducted at a constant current od 25-30 mA for 1.5 to 2 h. Following SDS-PAGE, the polyacrylamide gels were subjected to immunoblotting proteins by electrophoretically transferred in to a nitrocellulose membrane (Amersham Biosciences, Little Chalfon, UK) via Wetblotting at 100V (-300 mA) under cooling conditions for 1.5 to 2 h. Afterwards, membranes were blocked with 5% (w/v) non-fat dry milk in TBS-T or BSA on a rotary shaker at RT for 1 h. After incubation, membranes were washed 3 times with TBS-T for 10 min each. For detection of CDK9 proteins, membranes were incubated with a CDK9 antibody from Cell Signaling Technology (Danvers, MA, USA) at 1:1000 dilution overnight at 4°C. For a standard control an anti-tubulin antibody (Sigma-Aldrich) was used in a dilution of 1:10,000 for 1 h at RT. Then, membranes were washed 6 times with TBS-T on a rotary shaker at RT, 10 min each. Next, membranes were incubated with required secondary HRP-conjugated antibody at RT for 1 h. Subsequently, membranes were washed 3 times with TBS-T for 10 min. Finally, protein bands were detected with ECL substrate solution in a Vilber Lourmat chemiluminescence detection system (Software, Chemi-Capt 5000).

Determination of apoptosis

Apoptotic cell death was examined by DNA fragmentation according to the method of Nicoletti [28]. Infected and mock cells were collected by centrifugation at 1,500 rpm for 5 min, washed with 200 $\mu$l PBS and centrifuged again at 1,500 rpm for 5 min. Then, cells were lysed in 200 $\mu$l of Nicoletti lysis buffer (0.1% (w/v) sodium citrate pH 7.4, 0.1% (v/v) Triton X-100, 50 $\mu$g/ml propidium iodide) and stored overnight at 4°C in the dark. The propidium iodide stained DNA fragments were quantified by flow cytometry (FACS Canto II, Becton Dickinson). Results are presented as % specific DNA fragmentation according to the following formula:

```
(% experimental DNA fragmentation - % spontaneous DNA
fragmentation) /(100 - % spontaneous DNA fragmentation) x 100.
```

Cytotoxicity assay

Cytotoxicity was determined using the ApoTox-GloTM Triplex Assay kit (Promega, Mannheim, Germany). The live-cell protease activity is restricted to intact viable cells and is measured using a fluorogenic, cell-impermeant peptide substrate (bis-alanylalanyl-phenylalanyl-rhodamine 110; bis-AAF-R110) to measure dead-cell protease activity, which is released from cells that have lost membrane integrity. The assay was performed according to the manufacturer's instructions with the following changes:

different amounts (1250, 2500, 5000) of PC3 cells were seeded per well in a 96-well assay plate (Nunc™ MicroWell™ 96-Well-Platte) containing 100 $\mu$l of medium. Next day, cells were infected with the specific viruses in a 100 $\mu$l of medium for 1 h. Infected cells were cultured for additional 72, 96 and 120 h. Viability and cytotoxicity were measured at the selected time points by adding 20 $\mu$l per well of reagent containing both GF-AFC substrate and bis-AAF-R110 substrate to all wells, and briefly mix by orbital shaking (300-500 rpm for -30 seconds). Plates were incubated for 30 min at 37°C, and fluorescence was measured at the following two wavelength sets: 400Ex / 505 Em (Viability) and 485 Ex / 520 Em (Cytotoxicity).

In the same assay the caspase activity was detected by adding 100 $\mu$l per well of Caspase-Glo® 3/7 reagent to all wells (data not shown), and briefly mix by orbital shaking (300-500 rpm for -30 sec). After incubation of 1 to 2 h at room temperature, luminescence was measured (integration time 0.5-1 second).

Cell Viability Assay (*ATP levels)

Cell viability was also assessed in a 96-well format using the CellTiter-Glo® Luminescent Cell Viability Assay kit (Promega, Mannheim, Germany). This assay determines the number of viable cells by measuring the intracellular content of ATP. The amount of ATP correlates with the amount of metabolically active cells. The assay was performed according to the manufacturer's instructions with the following changes:

Different amounts (1250, 2500 and 5000) of PC3 cells were seeded per well in a 96-well assay plate (opaque-walled Nunc™ MicroWell™ 96-Well-Platte) containing 100 μl of medium. Next day, cells were infected with the selected virus in a 100 μl for 1 h. Infected cells were cultured for additional 72, 96 and 120 h. Viability was measured at the selected time points by carefully removing the medium and adding 100 μl of the CellTiter-Glo® reagent (1:1 diluted with medium). Then, plates were incubated in dark for 5 min on an orbital shaker at RT to induce cell lysis. Luminescence was measured using an Orion L Microplate Luminometer (Berthold Detection Systems) (integration time 1.0 sec).

Real-time detection of cell proliferation and viability

Cells were seeded at a density of $3 \times 10^3$ cells/well in a 96 well-E plate (ACEA Biosciences, San Diego, United States) in DMEM, supplemented with 5% FBS. On the next day, cells were infected with increasing MOIs of PVs or with virus dilution buffer (mock). Growth of mock treated cells and virus infected cells was monitored in real-time for 5-7 days using the xCELLigence System (ACEA Biosciences, San Diego, United States). Real time cell growth was expressed as normalized cell index (CI), a parameter directly proportional to the number of attached cells per well and, therefore, strictly correlated with cell proliferation rate. Cell proliferation was monitored in real time, every 30 min. The growth curves shown represent the averages of the results of three replicates with relative standard deviations.

Experiments with animal models

The experiments with the AsPC-1 xenograft rat model of human pancreatic carcinoma was performed as described in Li et al.[29] with the difference that virus treatment was carried out with $1 \times 10^8$ PFU/animal (with the exception of wild type H-1PV that was used at the at MOI of $1.25 \times 10^8$ PFU/animal) subdivided in 4 equal doses injected intratumourally at days 0, 7, 14 and 21.

**Example 2**

**Generation of ΔH-1PVshCDK9**

The inventors constructed pΔH-1PVshCDK9-a and pΔH-1PVshCDK9-b by inserting two shRNAs DNA sequences into the pΔH-1PVsilencer vector (Fig. 1)[19]. These sequences were selected through a small scale siRNA screening performed to identify siRNAs efficient in silencing the *CDK9* gene (data not shown). The selected siRNAs were then converted into shRNAs. The inventors first checked whether the insertion of the CDK9 shRNAs into ΔH-1PVsilencer was compatible with virus life cycle and fitness in one round of production. Typically, the first step of parvovirus production is carried out in HEK293T cells by transient transfection of the plasmid DNA harbouring the viral genome. At three days post-transfection, cells are harvested, lysed and new assembled progeny viral particles purified from lysates and titrated by real time qPCR and/or plaque assay. For virus amplification, NB324K cells are used as a packaging cell line. These cells are infected with low MOIs (ranging from 0.01 to 1 plaque forming unit/ml) of virus previously produced via DNA transfection in HEK293T cells. When cytopathic effects, indicator for virus replication and egress, are detected in 80% of the cells, cells are harvested and viral particles are purified and titrated as described above.

The pΔH-1PVshCDK9-a, pΔH-1PVshCDK9-b plasmids were transiently transfected in HEK-293T cells. As controls, the inventors used pSR19 (harbouring the wild type H-1PV genome [30]), pΔH-1PV (harbouring the ΔH-1PV genome featuring the 2022-2135 in frame deletion in the NS region) and pΔH-1PVshEGFP (containing the ΔH-1PV silencer genome including a shRNA sequence targeting EGFP). At three days after transfection, evident signs of cytopathic effects (CPE) were observed in all transfected cell-cultures indicating that virus production was taking place (data not shown). Consistently, viruses were successfully purified from cell lysates. No evident differences in the morphology of the various viruses were observed by electron microscopy (Fig. 2A). The size of the plaques obtained was also very similar (Fig. 2B). Virus yields were determined by q-PCR and plaque assays. According to previous results, ΔH-1PV and ΔH-1PVshEGFP were produced at higher levels than wild type H-1PV in HEK293T cells [18, 19] (Fig. 3A). Interestingly, differences in the production between the ΔH-1PVshCDK9-a and ΔH-1PVshCDK9-b were also noted with ΔH-1PVshCDK9-a produced at higher yields than ΔH-1PVshCDK9-b (Fig. 3A). In NB324K cells all viruses were produced at similar titers according to the quantification performed by qPCR (Fig. 3B light grey columns). However, these results were not entirely confirmed by plaque assays in which wild type H-1PV and ΔH-1PV were produced at higher levels than ΔH-1PVshEGFP and ΔH-1PVshCDK9-a (approximately 1 log difference). An even more dramatic reduction in production was observed for ΔH-1PVshCDK9-b (approximately two logs in comparison with ΔH-1PV) (Fig. 3B dark grey columns) suggesting that the insertion of this particular cassette impairs virus infectivity. Nevertheless these results indicate that it is possible to produce infectious PVs expressing shRNAs against the CDK9 gene.

**Example 3**

**ΔH-1PVshCDK9 is efficient in gene silencing**

In order to evaluate the silencing efficiency of ΔH-1PVshCDK9-a, PC3 cells were infected at MOI 1 and 5 PFU/cell of either ΔH-1PV, ΔH-1PVshEGFP, or ΔH-1PVshSCR or ΔH-1PVshCDK9-a. After 48 and 72 h post-infection, cells were harvested for total RNA and protein extraction respectively. qRT-PCR was carried out for analysing the steady-state levels of CDK9 mRNA. A strong reduction of the CDK9 mRNA levels was observed when cells were infected with the ΔH-1PVshCDK9 virus (Fig. 4A and data not shown). In agreement with these results, Western blot analysis on total cell lysates showed reduced CDK9 protein levels in ΔH-1PVshCDK9 infected cells, confirming that the ΔH-1PVshCDK9 has the ability to silence the *CDK9* gene expression (Fig. 4B).

**Example 4**

**ΔH-1PVshCDK9 viruses has enhanced oncotoxicity**

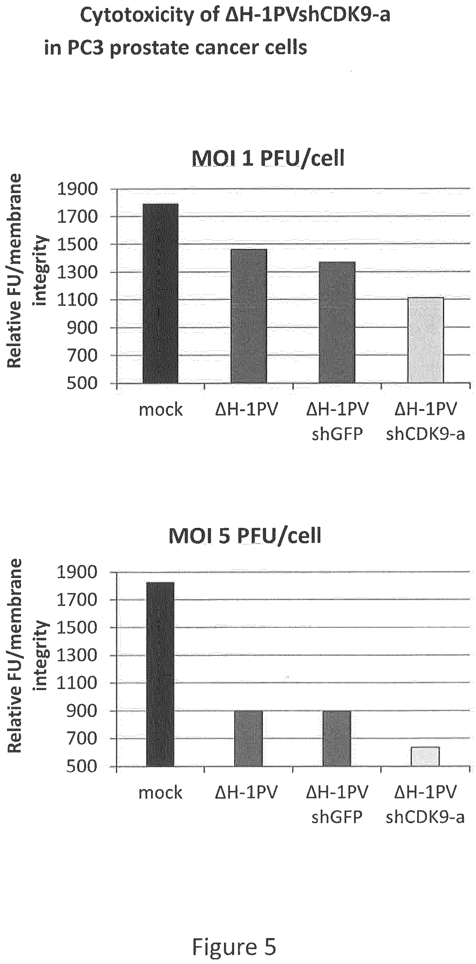

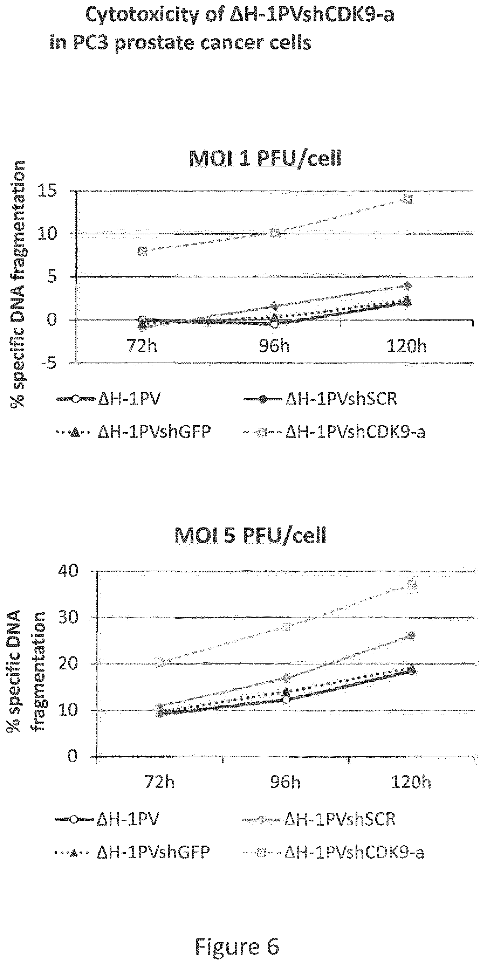

As a proof of concept, the PC3 prostate cancer cell line was used to evaluate the oncotoxic activity of ΔH-1PVshCDK9-a. To this end, PC3 cells were infected with increasing concentrations (MOI 1 and 5 PFU/cell) of ΔH-1PV, ΔH-1PVshSCR, ΔH-1PVshEGFP, or ΔH-1PVshCDK9-a or treated with virus-dilution buffer (mock). At three days post-infection virus-induced cytotoxicity was determined by analysing cell membrane integrity (virus induced cell lysis) using the ApoTox-Glo™ Triplex Assay kit. As shown in Fig. 5, increased cytotoxicity was observed in cells treated with ΔH-1PVshCDK9-a. Enhanced oncotoxicity of ΔH-1PVshCDK9-a was also confirmed by analysing the cells by flow cytometry for the detection of the sub-G1 apoptotic cell population containing fragmented DNA (Fig. 6). These results were further corroborated by cell viability (CellTiter-Glo® Luminescent Cell Viability Assay) and virus induced cell lysis (LDH assay) analyses (data not shown). In contrast, ΔH-1PVshCDK9-a did not affect peripheral blood nuclear cells from healthy donors, which are competent for virus entry, providing evidence that the ability to express shRNAs against CDK9 did not alter virus safety profile (data not shown).

Finally, the inventors used the xCELLigence technology to confirm the improved cytotoxicity of PV expressing shRNAs against CDK9. In addition to ΔH-1PVshCDK9a we also tested the cytotoxicity of ΔH-1PVshCDK9-b whose shRNA targets a different region of the CDK9 gene. As shown in Fig. 7, viruses expressing shRNAs against the CDK9 gene were more potent in killing cells than ΔH-1PV and ΔH-1PVshEGFP used at the same concentrations. Similar results were also obtained using another cancer cell line from pancreatic carcinoma, namely As-PC1 (Fig. 8). Altogether, these results demonstrated the superior oncolytic activity of ΔH-1PVshCDK9 viruses.

**Example 5**

**ΔH-1PVshCDK9 has improved oncosuppression activity**

The positive results obtained *in vitro,* prompted us to evaluate the oncosuppression activity of ΔH-1PVshCDK9 in an animal model of human cancer. To this end, we used the AsPC-1 xenograft nude rat model of human pancreatic carcinomas previously used in our laboratory [29].Tumour bearing animals were treated with PBS (mock), wild type H-1PV, ΔH-1PV, or ΔH-1PVshGFP or ΔH-1PVshCDK9-a at the concentration of $1 \times 10^8$ pfu/animal with the exception of wild type H-1PV that was used at the MOI of $1.25 \times 10^8$ pfu/animal (Fig. 9 A). We found that ΔH-1PV is more efficient than wild type H-1PV in delaying tumour growth, confirming previous results of the group. A further increase of the oncosuppressive ability was achieved with ΔH-1PV-shCDK9-a that at the concentration tested was significantly more efficient than ΔH-1PV and ΔH-1PV-shGFP in delaying tumour growth (P < 0.01) (Fig. 9B). The increase in oncotoxicity is also reflected by a significant improvement of the overall survival of the ΔH-1PV-shCDK9-a treated animals in comparison with other groups (Fig. 9C). No weight loss or other evident cytotoxic side effects were observed in virus treated animals. In conclusion, these results provide proof-of-concept that ΔH-1PVshCDK9-a has superior anticancer activity than wild type H-1PV or its ΔH-1PV natural variant warranting clinical translation of this virus in cancer patients.

**EP 3 327 124 A1**

**List of references**

1 Fire, A., Xu, S., Montgomery, M. K., Kostas, S. A., Driver, S. E. and Mello, C. C. (1998) Potent and specific genetic interference by double-stranded RNA in Caenorhabditis elegans. Nature. 391, 806-811

2 Davidson, B. L. and McCray, P. B., Jr. (2011) Current prospects for RNA interference-based therapies. Nat Rev Genet. 12, 329-340

3 Dorsett, Y. and Tuschl, T. (2004) siRNAs: applications in functional genomics and potential as therapeutics. Nat Rev Drug Discov. 3, 318-329

4 Rettig, G. R. and Behlke, M. A. (2012) Progress toward in vivo use of siRNAs-II. Molecular therapy : the journal of the American Society of Gene Therapy. 20, 483-512

5 Bobbin, M. L. and Rossi, J. J. (2016) RNA Interference (RNAi)-Based Therapeutics: Delivering on the Promise? Annual review of pharmacology and toxicology. 56, 103-122

6 Snove, O., Jr. and Rossi, J. J. (2006) Expressing short hairpin RNAs in vivo. Nat Methods. 3, 689-695

7 Brummelkamp, T. R., Bernards, R. and Agami, R. (2002) A system for stable expression of short interfering RNAs in mammalian cells. Science. 296, 550-553

8 Yoo, J. Y., Kim, J. H., Kwon, Y. G., Kim, E. C., Kim, N. K., Choi, H. J. and Yun, C. O. (2007) VEGF-specific short hairpin RNA-expressing oncolytic adenovirus elicits potent inhibition of angiogenesis and tumor growth. Mol Ther. 15, 295-302

9 Mao, L. J., Zhang, J., Liu, N., Fan, L., Yang, D. R., Xue, B. X., Shan, Y. X. and Zheng, J. N. (2015) Oncolytic virus carrying shRNA targeting SATB1 inhibits prostate cancer growth and metastasis. Tumour biology : the journal of the International Society for Oncodevelopmental Biology and Medicine. 36, 9073-9081

10 Jung, S. H., Choi, J. W., Yun, C. O., Yhee, J. Y., Price, R., Kim, S. H., Kwon, I. C. and Ghandehari, H. (2014) Sustained local delivery of oncolytic short hairpin RNA adenoviruses for treatment of head and neck cancer. J Gene Med. 16, 143-152

11 Fang, L., Cheng, Q., Li, W., Liu, J., Li, L., Xu, K. and Zheng, J. (2014) Antitumor activities of an oncolytic adenovirus equipped with a double siRNA targeting Ki67 and hTERT in renal cancer cells. Virus Res. 181, 61-71

12 Li, Y., Zhang, B., Zhang, H., Zhu, X., Feng, D., Zhang, D., Zhuo, B., Li, L. and Zheng, J. (2013) Oncolytic adenovirus armed with shRNA targeting MYCN gene inhibits neuroblastoma cell proliferation and in vivo xenograft tumor growth. Journal of cancer research and clinical oncology. 139, 933-941

13 Kim, J., Nam, H. Y., Kim, T. I., Kim, P. H., Ryu, J., Yun, C. O. and Kim, S. W. (2011) Active targeting of RGD-conjugated bioreducible polymer for delivery of oncolytic adenovirus expressing shRNA against IL-8 mRNA. Biomaterials. 32, 5158-5166

14 Gao, Y., Zhu, Y., Huang, X., Ai, K., Zheng, Q. and Yuan, Z. (2015) Gene therapy targeting hepatocellular carcinoma by a dual-regulated oncolytic adenovirus harboring the focal adhesion kinase shRNA. International journal of oncology. 47, 668-678

15 Zheng, J. N., Pei, D. S., Mao, L. J., Liu, X. Y., Mei, D. D., Zhang, B. F., Shi, Z., Wen, R. M. and Sun, X. Q. (2009) Inhibition of renal cancer cell growth in vitro and in vivo with oncolytic adenovirus armed short hairpin RNA targeting Ki-67 encoding mRNA. Cancer Gene Ther. 16, 20-32

16 Anesti, A. M., Simpson, G. R., Price, T., Pandha, H. S. and Coffin, R. S. (2010) Expression of RNA interference triggers from an oncolytic herpes simplex virus results in specific silencing in tumour cells in vitro and tumours in vivo. BMC cancer. 10, 486

17 Marchini, A., Bonifati, S., Scott, E. M., Angelova, A. L. and Rommelaere, J. (2015) Oncolytic parvoviruses: from basic virology to clinical applications. Virol J. 12, 6

18 Weiss, N., Stroh-Dege, A., Rommelaere, J., Dinsart, C. and Salome, N. (2012) An in-frame deletion in the NS protein-coding sequence of parvovirus H-1PV efficiently stimulates export and infectivity of progeny virions. J Virol. 86, 7554-7564

19 Illarionova, A., Rommelaere, J., Leuchs, B. and Marchini, A. ( 2012) Modified parvovirus useful for gene silencing. EP2620503

20 Morales, F. and Giordano, A. (2016) Overview of CDK9 as a target in cancer research. Cell Cycle. 15, 519-527

21 Wang, S. and Fischer, P. M. (2008) Cyclin-dependent kinase 9: a key transcriptional regulator and potential drug target in oncology, virology and cardiology. Trends Pharmacol Sci. 29, 302-313

22 Sims, R. J., 3rd, Belotserkovskaya, R. and Reinberg, D. (2004) Elongation by RNA polymerase II: the short and long of it. Genes Dev. 18, 2437-2468

23 Shapiro, G. I. (2006) Cyclin-dependent kinase pathways as targets for cancer treatment. J Clin Oncol. 24, 1770-1783

24 Romano, G. and Giordano, A. (2008) Role of the cyclin-dependent kinase 9-related pathway in mammalian gene expression and human diseases. Cell Cycle. 7, 3664-3668

25 Krystof, V., Baumli, S. and Furst, R. (2012) Perspective of cyclin-dependent kinase 9 (CDK9) as a drug target. Curr Pharm Des. 18, 2883-2890

26 Leuchs, B., Roscher, M., Muller, M., Kurschner, K. and Rommelaere, J. (2016) Standardized large-scale H-1PV production process with efficient quality and quantity monitoring. J Virol Methods. 229, 48-59

27 Laemmli, U. K. (1970) Cleavage of structural proteins during the assembly of the head of bacteriophage T4. Nature. 227, 680-685

28 Nicoletti, I., Migliorati, G., Pagliacci, M. C., Grignani, F. and Riccardi, C. (1991) A rapid and simple method for measuring thymocyte apoptosis by propidium iodide staining and flow cytometry. J Immunol Methods. 139, 271-279

29 Li, J., Bonifati, S., Hristov, G., Marttila, T., Valmary-Degano, S., Stanzel, S., Schnolzer, M., Mougin, C., Aprahamian, M., Grekova, S. P., Raykov, Z., Rommelaere, J. and Marchini, A. (2013) Synergistic combination of valproic acid and oncolytic parvovirus H-1PV as a potential therapy against cervical and pancreatic carcinomas. EMBO Mol Med. 5, 1537-1555

30 Kestler, J., Neeb, B., Struyf, S., Van Damme, J., Cotmore, S. F., D'Abramo, A., Tattersall, P., Rommelaere, J., Dinsart, C. and Cornelis, J. J. (1999) cis requirements for the efficient production of recombinant DNA vectors based on autonomous parvoviruses. Hum Gene Ther. 10, 1619-1632

**Claims**

1. Parvovirus for down regulating the expression of cyclin dependent kinase 9 (CDK9) in a cell **characterized in that** it is derived from a parvovirus H-1 deletion variant containing a deletion encompassing the nucleotides 2022-2135, wherein a CDK9 specific nucleic acid is inserted in an untranslated region downstream of the H-1 parvovirus VP gene and is expressible under the control of a promoter or promoter region recognizable by an RNA polymerase in the cell, wherein said CDK9 specific nucleic acid is transcribable in an RNAi, and wherein said parvovirus is capable of replicating and propagating in the cell.

2. The parvovirus of claim 1, wherein the CDK9 specific nucleic acid is inserted at nucleotide 4683 of the H-1PV genome.

3. The parvovirus of claim 1 or 2, wherein the promoter or promoter region recognizable by a RNA polymerase of the cell is an RNA-polymerase II (Pol II) or III (Pol III) promoter.

4. The parvovirus of claim 3, wherein the RNA-polymerase III (Pol III) promoter is the RNA-polymerase III H1 promoter.

5. The parvovirus of any one of claims 1 to 4, wherein the target specific nucleic acid is an shRNA.

6. The parvovirus of any one of claims 1 to 5, wherein the target specific nucleic acid has a length of at least 15 nucleotides.

7. The parvovirus according to any one of claims 1 to 6 for the use in a method of treating a tumour.

8. The parvovirus for the use according to claim 7 **characterized in that** the cells of said tumour are resistant to chemotherapy and/or radiotherapy.

9. The parvovirus for the use according to claim 7 or 8, **characterized in that** said parvovirus is administered by intravenous (i.v.), intratumoral or endobronchial administration.

10. A cell containing a parvovirus of any one of claims 1 to 6.

11. A transgenic non-human animal comprising a cell of claim 10.

**ΔH-1PVsilencer**

Figure 1

A

## Electron microscopy

H-1PV        ΔH-        ΔH-1PVshEGFP

ΔH-1PVshCDK9-a        ΔH-1PVshCDK9-

B

## Plaque assay

H-1PV        ΔH-1PV        ΔH-1PVshEGFP

ΔH-1PVshCDK9-a        ΔH-1PVshCDK9-b

Figure 2

A

B

Figure 3

# Silencing efficacy of ΔH-1PVshCDK9-a
# in PC3 prostate cancer cells

Figure 4

# Cytotoxicity of ΔH-1PVshCDK9-a
# in PC3 prostate cancer cells

Figure 5

## Cytotoxicity of ΔH-1PVshCDK9-a
## in PC3 prostate cancer cells

Figure 6

## Cytotoxicity of ΔH-1PVshCDK9
## in PC3 prostate cancer cells

Figure 7

# Cytotoxicity of ΔH-1PVshCDK9
# in AsPC-1 pancreatic carcinoma cells

Figure 8

9A

9B

Figure 9 A-B

C

Kaplan-Meier survival curves

Figure 9 C

# EP 3 327 124 A1

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 2 620 503 A1 (DEUTSCHES KREBSFORSCH [DE]) 31 July 2013 (2013-07-31) * Whole doc., in particular para. [0031, 0034] * | 1-11 | INV. C12N15/113 C12N15/864 |
| Y | CHUN-HAO HUANG ET AL: "CDK9-mediated transcription elongation is required for MYC addiction in hepatocellular carcinoma", GENES AND DEVELOPMENT., vol. 28, no. 16, 15 August 2014 (2014-08-15), pages 1800-1814, XP55229752, US ISSN: 0890-9369, DOI: 10.1101/gad.244368.114 * Whole doc., in particular p.1802, 1807 * | 1-11 | |
| Y | J LEMKE ET AL: "Selective CDK9 inhibition overcomes TRAIL resistance by concomitant suppression of cFlip and Mcl-1", CELL DEATH AND DIFFERENTIATION., vol. 21, no. 3, 1 March 2014 (2014-03-01), pages 491-502, XP55346725, GB ISSN: 1350-9047, DOI: 10.1038/cdd.2013.179 * Whole doc., in particular p.494, col.2; Fig.5 * | 1-11 | TECHNICAL FIELDS SEARCHED (IPC)  C12N |
| Y | WO 2004/042002 A2 (UNIV MASSACHUSETTS [US]; RANA TARIQ M [US]) 21 May 2004 (2004-05-21) * Whole doc., in particular p.48-53, 72; Fig.3a; Example 5 * | 1-11 | |

-/--

The present search report has been drawn up for all claims

| Place of search | Date of completion of the search | Examiner |
|---|---|---|
| Munich | 17 February 2017 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 16 20 0978

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GAETANO ROMANO: "Deregulations in the Cyclin-Dependent Kinase-9-Related Pathway in Cancer: Implications for Drug Discovery and Development", ISRN ONCOLOGY, vol. 2013, 1 January 2013 (2013-01-01), pages 1-14, XP55346739, ISSN: 2356-7872, DOI: 10.1155/2013/305371 * the whole document * ----- | 1-11 | |

TECHNICAL FIELDS
SEARCHED      (IPC)

The present search report has been drawn up for all claims

| Place of search | Date of completion of the search | Examiner |
|---|---|---|
| Munich | 17 February 2017 | Roscoe, Richard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 16 20 0978

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2017

| Patent document cited in search report | | Publication date | Patent family member(s) | | Publication date |
|---|---|---|---|---|---|
| EP 2620503 | A1 | 31-07-2013 | AU 2013211755 | A1 | 27-03-2014 |
| | | | CA 2849370 | A1 | 01-08-2013 |
| | | | DK 2620503 | T3 | 15-12-2014 |
| | | | EP 2620503 | A1 | 31-07-2013 |
| | | | EP 2807259 | A1 | 03-12-2014 |
| | | | ES 2526154 | T3 | 07-01-2015 |
| | | | JP 5901796 | B2 | 13-04-2016 |
| | | | JP 2015506178 | A | 02-03-2015 |
| | | | US 2014310829 | A1 | 16-10-2014 |
| | | | WO 2013110464 | A1 | 01-08-2013 |
| WO 2004042002 | A2 | 21-05-2004 | AU 2003299531 | A1 | 07-06-2004 |
| | | | US 2004204420 | A1 | 14-10-2004 |
| | | | WO 2004042002 | A2 | 21-05-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4873191 A

- EP 2620503 A

**Non-patent literature cited in the description**

- **FINGL et al.** The Pharmocological Basis of Therapeutics. Macmillan Publishing Co, 1975, 1-46
- **FIRE, A. ; XU, S. ; MONTGOMERY, M. K. ; KOSTAS, S. A. ; DRIVER, S. E. ; MELLO, C. C.** Potent and specific genetic interference by double-stranded RNA in Caenorhabditis elegans. *Nature,* 1998, vol. 391, 806-811
- **DAVIDSON, B. L. ; MCCRAY, P. B., JR.** Current prospects for RNA interference-based therapies. *Nat Rev Genet.,* 2011, vol. 12, 329-340
- **DORSETT, Y. ; TUSCHL, T.** siRNAs: applications in functional genomics and potential as therapeutics. *Nat Rev Drug Discov.,* 2004, vol. 3, 318-329
- **RETTIG, G. R. ; BEHLKE, M. A.** Progress toward in vivo use of siRNAs-II. *Molecular therapy : the journal of the American Society of Gene Therapy,* 2012, vol. 20, 483-512
- **BOBBIN, M. L. ; ROSSI, J. J.** RNA Interference (RNAi)-Based Therapeutics: Delivering on the Promise?. *Annual review of pharmacology and toxicology,* 2016, vol. 56, 103-122
- **SNOVE, O., JR. ; ROSSI, J. J.** Expressing short hairpin RNAs in vivo. *Nat Methods,* 2006, vol. 3, 689-695
- **BRUMMELKAMP, T. R. ; BERNARDS, R. ; AGAMI, R.** A system for stable expression of short interfering RNAs in mammalian cells. *Science,* 2002, vol. 296, 550-553
- **YOO, J. Y. ; KIM, J. H. ; KWON, Y. G. ; KIM, E. C. ; KIM, N. K. ; CHOI, H. J. ; YUN, C. O.** VEGF-specific short hairpin RNA-expressing oncolytic adenovirus elicits potent inhibition of angiogenesis and tumor growth. *Mol Ther.,* 2007, vol. 15, 295-302
- **MAO, L. J. ; ZHANG, J. ; LIU, N. ; FAN, L. ; YANG, D. R. ; XUE, B. X. ; SHAN, Y. X. ; ZHENG, J. N.** Oncolytic virus carrying shRNA targeting SATB1 inhibits prostate cancer growth and metastasis. *Tumour biology : the journal of the International Society for Oncodevelopmental Biology and Medicine,* 2015, vol. 36, 9073-9081

- **JUNG, S. H. ; CHOI, J. W. ; YUN, C. O. ; YHEE, J. Y. ; PRICE, R. ; KIM, S. H. ; KWON, I. C. ; GHANDEHARI, H.** Sustained local delivery of oncolytic short hairpin RNA adenoviruses for treatment of head and neck cancer. *J Gene Med.,* 2014, vol. 16, 143-152
- **FANG, L. ; CHENG, Q. ; LI, W. ; LIU, J. ; LI, L. ; XU, K. ; ZHENG, J.** Antitumor activities of an oncolytic adenovirus equipped with a double siRNA targeting Ki67 and hTERT in renal cancer cells. *Virus Res.,* 2014, vol. 181, 61-71
- **LI, Y. ; ZHANG, B. ; ZHANG, H. ; ZHU, X. ; FENG, D. ; ZHANG, D. ; ZHUO, B. ; LI, L. ; ZHENG, J.** Oncolytic adenovirus armed with shRNA targeting MYCN gene inhibits neuroblastoma cell proliferation and in vivo xenograft tumor growth. *Journal of cancer research and clinical oncology,* 2013, vol. 139, 933-941
- **KIM, J. ; NAM, H. Y. ; KIM, T. I. ; KIM, P. H. ; RYU, J. ; YUN, C. O. ; KIM, S. W.** Active targeting of RGD-conjugated bioreducible polymer for delivery of oncolytic adenovirus expressing shRNA against IL-8 mRNA. *Biomaterials,* 2011, vol. 32, 5158-5166
- **GAO, Y. ; ZHU, Y. ; HUANG, X. ; AI, K. ; ZHENG, Q. ; YUAN, Z.** Gene therapy targeting hepatocellular carcinoma by a dual-regulated oncolytic adenovirus harboring the focal adhesion kinase shRNA. *International journal of oncology,* 2015, vol. 47, 668-678
- **ZHENG, J. N. ; PEI, D. S. ; MAO, L. J. ; LIU, X. Y. ; MEI, D. D. ; ZHANG, B. F. ; SHI, Z. ; WEN, R. M. ; SUN, X. Q.** Inhibition of renal cancer cell growth in vitro and in vivo with oncolytic adenovirus armed short hairpin RNA targeting Ki-67 encoding mRNA. *Cancer Gene Ther.,* 2009, vol. 16, 20-32
- **ANESTI, A. M. ; SIMPSON, G. R. ; PRICE, T. ; PANDHA, H. S. ; COFFIN, R. S.** Expression of RNA interference triggers from an oncolytic herpes simplex virus results in specific silencing in tumour cells in vitro and tumours in vivo. *BMC cancer,* 2010, vol. 10, 486

- **MARCHINI, A. ; BONIFATI, S. ; SCOTT, E. M. ; ANGELOVA, A. L. ; ROMMELAERE, J.** Oncolytic parvoviruses: from basic virology to clinical applications. *Virol J.,* 2015, vol. 12, 6
- **WEISS, N. ; STROH-DEGE, A. ; ROMMELAERE, J. ; DINSART, C. ; SALOME, N.** An in-frame deletion in the NS protein-coding sequence of parvovirus H-1PV efficiently stimulates export and infectivity of progeny virions. *J Virol.,* 2012, vol. 86, 7554-7564
- **ILLARIONOVA, A. ; ROMMELAERE, J. ; LEUCHS, B. ; MARCHINI, A.** *Modified parvovirus useful for gene silencing,* 2012
- **MORALES, F. ; GIORDANO, A.** Overview of CDK9 as a target in cancer research. *Cell Cycle,* 2016, vol. 15, 519-527
- **WANG, S. ; FISCHER, P. M.** Cyclin-dependent kinase 9: a key transcriptional regulator and potential drug target in oncology, virology and cardiology. *Trends Pharmacol Sci.,* 2008, vol. 29, 302-313
- **SIMS, R. J., 3RD ; BELOTSERKOVSKAYA, R. ; REINBERG, D.** Elongation by RNA polymerase II: the short and long of it. *Genes Dev.,* 2004, vol. 18, 2437-2468
- **SHAPIRO, G. I.** Cyclin-dependent kinase pathways as targets for cancer treatment. *J Clin Oncol.,* 2006, vol. 24, 1770-1783
- **ROMANO, G. ; GIORDANO, A.** Role of the cyclin-dependent kinase 9-related pathway in mammalian gene expression and human diseases. *Cell Cycle,* 2008, vol. 7, 3664-3668
- **KRYSTOF, V. ; BAUMLI, S. ; FURST, R.** Perspective of cyclin-dependent kinase 9 (CDK9) as a drug target. *Curr Pharm Des.,* 2012, vol. 18, 2883-2890
- **LEUCHS, B. ; ROSCHER, M. ; MULLER, M. ; KURSCHNER, K. ; ROMMELAERE, J.** Standardized large-scale H-1PV production process with efficient quality and quantity monitoring. *J Virol Methods,* 2016, vol. 229, 48-59
- **LAEMMLI, U. K.** Cleavage of structural proteins during the assembly of the head of bacteriophage T4. *Nature,* 1970, vol. 227, 680-685
- **NICOLETTI, I. ; MIGLIORATI, G. ; PAGLIACCI, M. C. ; GRIGNANI, F. ; RICCARDI, C.** A rapid and simple method for measuring thymocyte apoptosis by propidium iodide staining and flow cytometry. *J Immunol Methods,* 1991, vol. 139, 271-279
- **LI, J. ; BONIFATI, S. ; HRISTOV, G. ; MARTTILA, T. ; VALMARY-DEGANO, S. ; STANZEL, S. ; SCHNOLZER, M. ; MOUGIN, C. ; APRAHAMIAN, M. ; GREKOVA, S. P.** Synergistic combination of valproic acid and oncolytic parvovirus H-1PV as a potential therapy against cervical and pancreatic carcinomas. *EMBO Mol Med.,* 2013, vol. 5, 1537-1555
- **KESTLER, J. ; NEEB, B. ; STRUYF, S. ; VAN DAMME, J. ; COTMORE, S. F. ; D'ABRAMO, A. ; TATTERSALL, P. ; ROMMELAERE, J. ; DINSART, C. ; CORNELIS, J. J.** cis requirements for the efficient production of recombinant DNA vectors based on autonomous parvoviruses. *Hum Gene Ther.,* 1999, vol. 10, 1619-1632